# EUROPEAN PATENT SPECIFICATION

(11) **EP 3 273 917 B1**
(45) Date of publication and mention of the grant of the patent: **12.02.2020**
(21) Application number: 16767592.5
(22) Date of filing: 23.03.2016
(51) Int. Cl.: A61F 2/46, A61B 17/88, A61B 17/90, A61F 2/40

(54) **GLENOSPHERE-IMPLANT POSITIONING DEVICE**
GLENOSPHÄRENIMPLANTATPOSITIONIERUNGSVORRICHTUNGEN
DISPOSITIF DE POSITIONNEMENT D'IMPLANT DE GLÉNOSPHÈRE

(30) Priority: 25.03.2015 US 201562138175 P; 26.03.2015 US 201562138746 P
(43) Date of publication of application: 31.01.2018
(73) Proprietor: Orthosoft ULC, Montréal QC H3C 2N6 (CA)
(72) Inventor: MÉRETTE, Jean-Sébastien, Mont-St-Hilaire, Québec J3H 4W2 (CA); NEUROHR, Anselm Jakob, Montréal, Québec H2W 2B7 (CA); VAN KAMPEN, William, Saline, Michigan 48176 (US)
(74) Representative: Taor, Simon Edward William
(86) International application number: PCT/CA2016/050338
(87) International publication number: WO 2016/149825

(56) References cited:
- US-A1- 2006 074 430
- US-A1- 2012 059 383
- US-A1- 2012 143 267
- US-A1- 2014 107 715
- US-A1- 2015 073 424
- US-B2- 8 900 245

## Description

### FIELD OF THE APPLICATION

The present application relates to the glenoid implant surgery using patient specific instrumentation or like assistance devices.

### BACKGROUND OF THE ART

In orthopedic shoulder surgery, implant components are installed on the glenoid portion of the scapula (i.e., shoulder blade) and/or on the humerus, to replicate the shoulder joint. When an implant is installed on the scapula, it is commonly installed in the glenoid cavity, also known as the glenoid or glenoid fossa. The glenoid is a cavity that receives the head of the humerus in an anatomical shoulder. When an implant is used with the glenoid, a base of the implant is located within the glenoid, and could be secured thereto by fasteners such as screws, or using cement and/or fixation peg or keel.

One of the challenges in the case of shoulder surgery is with the placement of a glenosphere of the implant. Indeed, an improperly placed glenosphere could impact natural movements of the arm relative to the shoulder and require a premature revision surgery.

Patient specific instrumentation (hereinafter "PSI") pertains to the creation of instruments that are made specifically for the patient. PSI are typically manufactured from data using imagery to model bone geometry. Therefore, PSI have surfaces that may contact the bone in a predictable way as such contact surfaces are specifically manufactured to match the surface of a bone. It would therefore be desirable to use PSI technology in shoulder surgery. US Application Publication No. US 2015/073424 A1 discloses a method for resurfacing a glenoid in which PSI technology for placing a pin in a bone (e.g., the glenoid) with at least two pin slots may be used. US Application Publication No. US 2015/073424 A1 further discloses how such pin, once placed in the bone, may be used for positioning the base relative to the bone. On the other hand, difficulties remain with the positioning of the glenosphere due for example to its hemispherical shape. It would be desirable to provide assistance in the positioning of the glenosphere.

### SUMMARY OF THE APPLICATION

It is therefore an aim of the present invention to provide a patient specific glenosphere implant positioning device for assisting in implant the glenosphere implant.

Therefore, in accordance with a first embodiment of the present disclosure, there is provided a glenosphere-implant positioning device comprising: a coupling body having at least a first portion adapted to contact a spherical portion of a glenosphere implant, and a second portion adapted to contact an underside portion of the glenosphere implant, such that the coupling body is configured to be releasably coupled to a glenosphere implant in a known manner; and a guide connected to the coupling body and configured to be slidingly engaged to a guide pin representative of a desired implanting orientation of the glenosphere implant or to a periphery of an implanted baseplate.

Further in accordance with the first embodiment, wherein the coupling body has a C shape.

Still further in accordance with the first embodiment, the first portion of coupling body is an arcuate member having an inward sphere-matching surface configured to conformly contact the spherical portion of a glenosphere implant.

Still further in accordance with the first embodiment, the second portion is a flange projecting inwardly from an edge of the arcuate member.

Still further in accordance with the first embodiment, the guide is an edge surface of the flange configured to form a sliding joint with the periphery of the implanted baseplate.

Still further in accordance with the first embodiment, the C shape is configured to be releasably coupled over more than 180 degrees of the glenosphere implant.

Still further in accordance with the first embodiment, the guide defines a channel configured to be slidingly engaged to a guide pin, the glenosphere-implant positioning device further comprising a patient specific geometry between the coupling body and the channel based on a planned position and orientation between the guide pin and a baseplate upon which the glenosphere implant is to be implanted.

Still further in accordance with the first embodiment, at least one connector projects from the coupling body and configured for releasable engagement with an impactor tool interface contacting the glenosphere-implant.

Still further in accordance with the first embodiment, the at least one connector is configured to align the impact tool portion with the desired implanting orientation.

Still further in accordance with the first embodiment, the glenosphere-implant positioning device is a monolithic component.

In accordance with a second embodiment of the present disclosure, there is provided a kit comprising: the glenosphere-implant positioning device as described above; the baseplate configured to be anchored to a bone; and the glenosphere implant for mating engagement with the baseplate, the glenosphere implant configured to be releasably coupled to the glenosphere-implant positioning device in a known manner.

Further in accordance with the second embodiment, an impactor tool interface is adapted to be positioned directly against a surface of the glenosphere implant.

Still further in accordance with the second embodiment, the impactor tool interface is adapted to be releasably connected to the glenosphere-implant positioning device such that an axis of an impactor tool is parallel to a normal to a plane of the baseplate.

The invention may be used in a method for installing a glenosphere implant on an implanted baseplate, comprising: releasably coupling the glenosphere implant to glenosphere-implant positioning device; forming a translational joint between the glenosphere-implant positioning device and one of an implanted guide pin and the baseplate, the translational joint being in a fixed desired orientation relative to the implanted baseplate; moving the glenosphere implant releasably coupled to glenosphere-implant positioning device along the fixed desired orientation into engagement with the baseplate; and impacting the glenosphere implant onto the baseplate along the fixed desired orientation.

Further in accordance with the method, forming a translational joint comprises sliding a guide channel of the glenosphere-implant positioning device onto the guide pin.

Still further in accordance with the method, forming a translational joint comprises for adding a rotational degree of freedom to the translation joint.

Still further in accordance with the method, forming a translational joint comprises displacing an edge surface of the glenosphere-implant positioning device into engagement with a circumferential surface of the implanted baseplate.

Still further in accordance with the method, further comprising releasably connecting an impactor tool portion to the glenosphere-implant positioning device and against the glenosphere implant prior to impacting.

Still further in accordance with the method, releasably connecting an impactor tool portion comprises aligning the impactor tool portion with the fixed desired orientation.

Still further in accordance with the method, wherein releasably coupling the glenosphere implant to glenosphere-implant positioning device comprises elastically derforming the glenosphere-implant positioning device during coupling.

### BRIEF DESCRIPTION OF THE DRAWINGS

Fig. 1 is an assembly view of a patient-specific glenosphere-implant positioning device coupled to a glenosphere implant and to a guide pin, relative to a baseplate;
Fig. 2 is a perspective view showing use of the glenosphere-implant positioning device, outside of an incision;
Fig. 3 is a perspective view showing use of the glenosphere-implant positioning device, within an incision;
Fig. 4 is a perspective view of the glenosphere-implant positioning device coupled to the glenosphere implant as in Fig. 1, with an impactor tool interface;
Fig. 5A is a perspective view of another embodiment of the patient-specific glenosphere-implant positioning device, with connectors for interfacing with an impactor tool interface;
Fig. 5B is a plan view of the glenosphere-implant positioning device of Fig. 5A;
Fig. 5C is a perspective view of the glenosphere-implant positioning device of Fig. 5A, as coupled to the glenosphere implant and to the impactor tool interface;
Fig. 5D is a side view of the glenosphere-implant positioning device of Fig. 5A, as coupled to the glenosphere implant, to the impactor tool interface and to a baseplate;
Fig. 6 is a perspective view of yet another embodiment of the patient-specific glenosphere-implant positioning device, as coupled to a glenosphere implant;
Fig. 7A is a perspective view of another embodiment of the glenosphere-implant positioning device, for guiding engagement with a baseplate;
Fig. 7B is a plan view of the glenosphere-implant positioning device of Fig. 7A;
Fig. 7C is a perspective view of the glenosphere-implant positioning device of Fig. 7A, as coupled to the glenosphere implant; and
Fig. 7D is a side view of the glenosphere-implant positioning device of Fig. 7A, as coupled to the glenosphere implant and to the baseplate.

### DESCRIPTION OF THE EXEMPLARY EMBODIMENTS

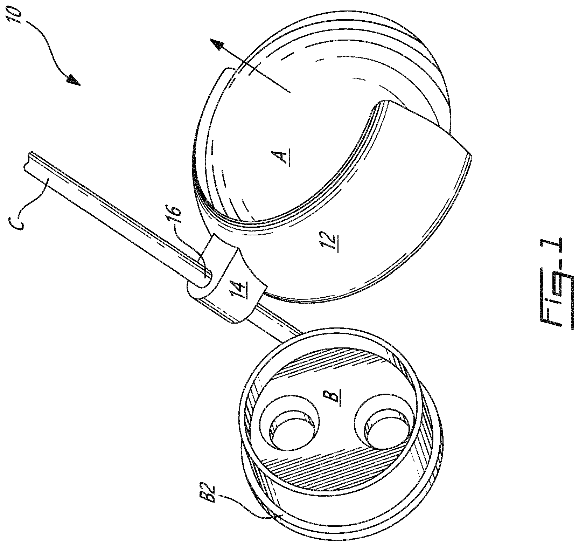

Referring to the drawings and more particularly to Fig. 1, there is illustrated at 10 a patient specific (PSI) glenosphere implant positioning device, for positioning a glenosphere implant A on implanted baseplate B, using a guide pin C. The implanted baseplate B and guide pin C have already been installed when the device 10 is used, their installation using for example techniques described in PCT Application Publication No. WO 2013/1429989. Other techniques could also be used, as that of PCT Application Publication No. WO 2013/1429989 is only provided as an example. According to the method for securing a glenoid implant on a scapula (i.e., scapula) in the PCT publication, patient specific instrumentation of various kinds may be used. The glenosphere may also be known as a glenoid hemispherical head implant base, in reverse total shoulder surgery.

The positioning device 10 has a coupling body 12 designed to be releasably coupled to the glenosphere implant A, in a known manner, such that a position of an axis of the glenosphere implant A (for instance a normal to a plane of its underside) is known relative to the coupling body 12. The positioning device 10 further comprises a guide 14. The guide 14 has a channel 16 that is slidably mountable on the guide pin C. Therefore, the assembly of the guide pin C and guide 14 forms a translational joint along the guide pin C, i.e., along a longitudinal axis of the guide pin C. Moreover, when the guide pin C has a circular section as in Fig. 1, a rotational joint may be present. In the embodiment of Fig. 1, the assembly of the guide pin C and guide 14 provides one translational degree of freedom (DOF), and one rotational DOF. Some play could be added to the connection design to compensate for potential slight misalignment between the pin and the baseplate B axis. Although a single guide 14 is shown, it is contemplated to have a pair of guides 14, to facilitate the use of the positioning device 10 on either the right shoulder or left shoulder.

The positioning device 10 may be said to be patient specific, in that the size of the coupling body 12, and/or the distance and orientation between the coupling body 12 and the channel 16 may result from patient specific data obtained preoperatively or intraoperatively, for example as in described in the above-referred PCT application. Stated differently, the position and orientation of the guide pin C relative to the baseplate B has been planned and replicates the planning, and the positioning device 10 is created as a function of the planned relation between the baseplate B and guide pin C.

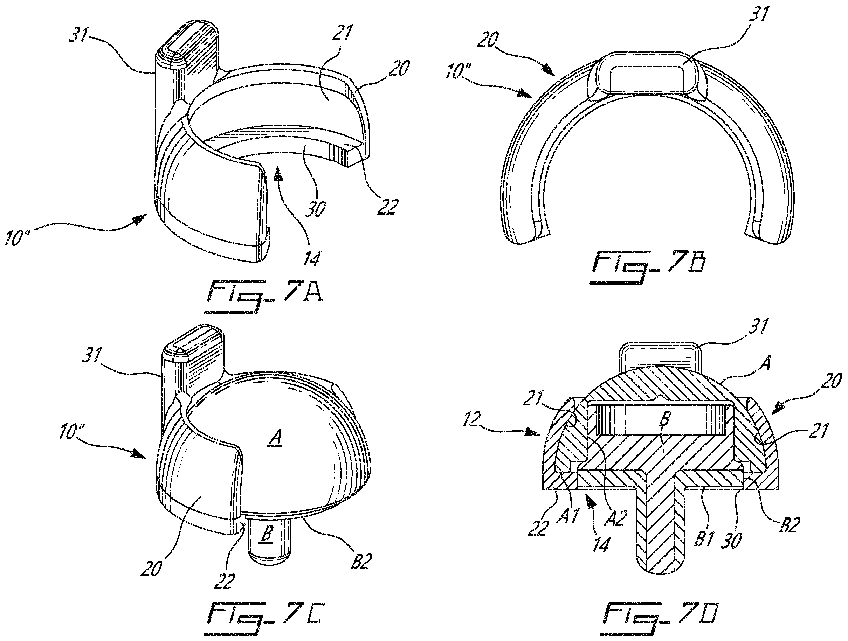

As shown in Fig. 7D, the glenosphere implant A may have an underside A1 featuring a shoulder sharing an edge with the hemi-spherical portion, and a socket A2 among numerous other possibilities, so as to be matingly engaged to a projecting connector of the baseplate B. In the illustrated embodiment, the projecting connector of baseplate B tapers toward the implant A, for instance by way of a Morse taper, to facilitate mating engagement between the implant A and the baseplate B. The positioning device 10 is created to ensure that the implant A sits on the baseplate B in a predicted and desired manner, for instance by having an axis of the implant A in a substantially normal relation to a plane B1 of the baseplate B. A normal of the plane B1 may be regarded as a desired orientation that serves as guide for the connection of the implant A to the baseplate B. The axis of the guide pin C is in a fixed relation with this desired orientation, and may be substantially parallel to it.

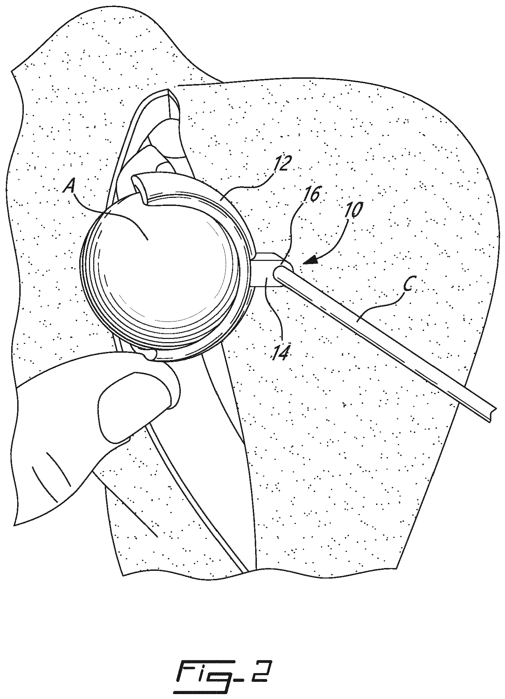

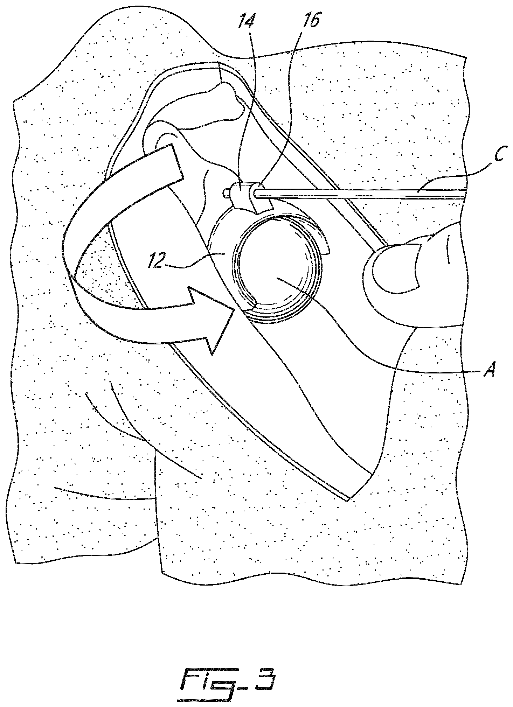

Referring to Figs. 2 and 3, a method for using the device 10 is illustrated, relative to a shoulder. The baseplate B and the guide pin C are anchored to the scapula based on the teachings of the above-referred PCT publication, or according to any other appropriate method. The guide pin C has a known orientation relative to that of the baseplate B. In an embodiment, an axis of the guide pin C is parallel to a normal of the plane B1 or the baseplate B, and thus to a desired installation axis of the implant A. The implant A is coupled to the coupling body 12 of the device 10, and the guide 14 is slid onto the guide pin C that projects out of the incision. The device 10 may be rotated about the longitudinal axis of the guide pin C, for the assembly of the coupling body 12 and the implant A to then be inserted through the incision by sliding the assembly along the guide pin C. The rotation may help orienting the assembly to a favorable orientation in light of the size constraints of the soft tissue in which the incision is made.

The assembly may be moved further along the guide pin C and rotated until the implant A is substantially aligned with the baseplate B. The complementary coupling between the implant A and the baseplate B will assist the operator in finding the appropriate alignment, and the constraints of the translation joint - i.e., the guide 14 on the guide pin C - will preserve the desired orientation of the implant A relative to the baseplate B. Hence, the guide pin C ensures that the axis of the implant A is oriented as planned relative to the baseplate B.

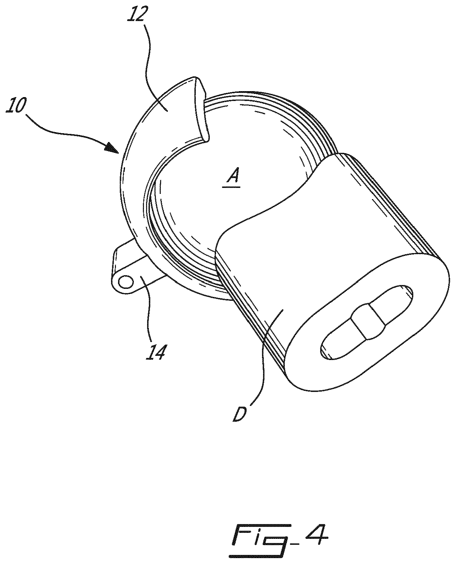

Once this achieved, the implant A may be secured to the baseplate B, for instance by impacting. As shown in Fig. 4, an impactor tool interface D (e.g., part of the impactor tool) or an impactor tool head is illustrated as having an interface surface conforming to the surface of the implant A. Moreover, the C-shape of the coupling body 12 provides a clearance for the impactor tool interface D to contact the implant A directly. To remove the positioning device 10, the guide pin C is first removed, allowing the coupling body 12 to be laterally removed from coupling engagement with the implant A.

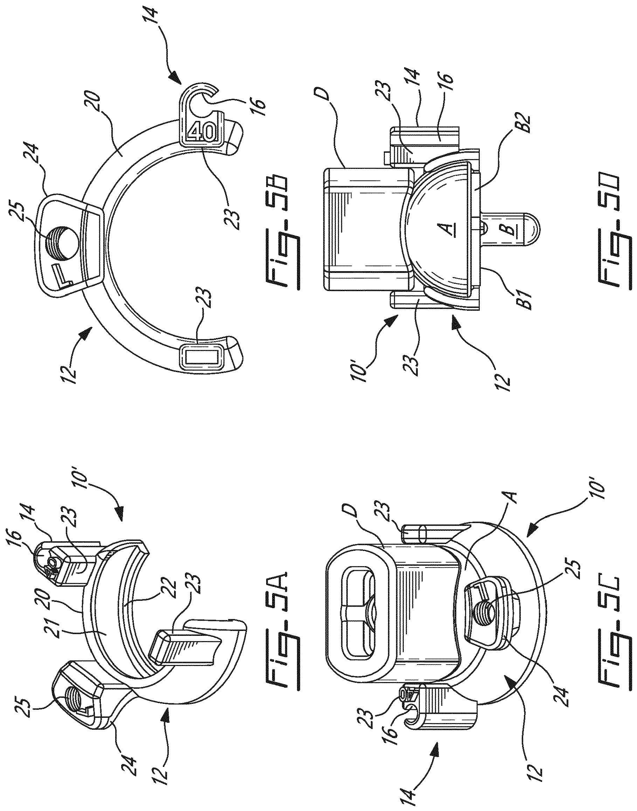

According to another embodiment shown in Figs. 5A to 5D, a positioning device 10' is shown in greater detail, and has many features in common with the positioning device 10 of Fig. 1, whereby like components will bear like reference numerals. The coupling body 12 of the positioning device 10' also has a C-shape, with a first portion being an arcuate member 20 being defined by a sphere-matching surface 21 forming a concavity for conforming contact with the frusto-spherical portion of the glenosphere implant A, as shown in Figs. 5C and 5D. A second portion may be an inward flange 22 located at a bottom edge of the arcuate member 20. The inward flange 22 abuts against the shoulder of the underside A1 of the glenosphere implant A, as seen in Fig. 5D. Therefore, the combination of the first portion and the second portion of the positioning device 10' allow it to be releasably coupled to the glenosphere implant A. To further assist in the releasable coupling between the positioning device 10' and the glenosphere implant A, the C shape of the coupling body 12 may surround the glenosphere implant A over more than 180 degrees. In Fig. 5B, the C shape is shown as being more than a half circle for this purpose, although a sufficient coupling may be achieved with 180 degrees or less (with no elastic deformation required). Hence, to releasably couple the positioning device 10' to the glenosphere implant A, the positioning device 10' is made of a material capable of elastic deformation when deformed to be coupled to the glenosphere implant A, such a medical-grade polymer or metal. In an embodiment, the positioning device 10/10' is a monolithic component. The positioning device 10' may be held captive against the glenosphere implant A such that they are handled as one component.

Connectors 23 may be provided on a top end of the coupling body 12. The connectors 23 are sized for receiving therebetween the end of the impactor tool, such as the impactor tool interface D. These connectors 23 may further assist in preserving the desired orientation of the implant A relative to the baseplate B, for instance by aligning the shaft of the impactor tool with the guide pin C, for visual assistance, and thus with the normal of the baseplate plane B1. The guide channel 14 may be formed in one of the connectors 23. The guide channel 14 may be laterally slotted to facilitate removal, although it is contemplated to suggest removal of the guide pin C prior to uncoupling the positioning device 10/10' from the implant A installed in the baseplate B.

A block 24 also projects upwardly from the coupling body 12. The block 24 has a threaded hole 25 for being connected to a rod of a manipulating tool. As observed in Figs. 2 and 3, the positioning device 10 and 10' may be handled by an operator's fingers. However, the positioning device 10 and 10' may optionally be manipulated by the assistance of a manipulating tool releasably connected to the positioning device 10' by the threaded hole 25. The block 24 may or may not be used as a connector for connecting the impactor tool to the positioning device 10', in similar fashion to the connectors 23. Although separate connectors 23, a single one could be present, for instance covering more or less 180 degrees to connect to both sides of the interface D.

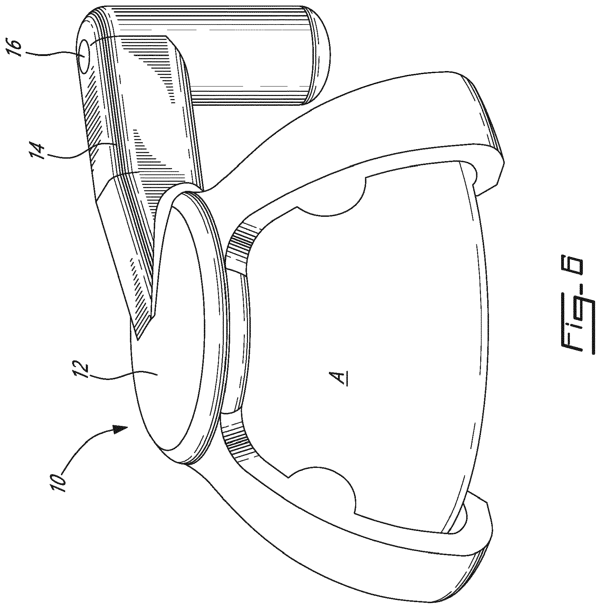

The coupling body 12 may have any appropriate shape to be coupled to the implant A. As the implant A has a hemispherical surface, the coupling body 12 may have sphere-matching surfaces. Fig. 6 shows an alternative embodiment, in which the coupling body 12 has a three-pronged configuration for grasping the implant A. The first portion of the coupling body is therefore defined by three contact pads, whereas the second portion features tabs contacting the undersurface A1 of the glenosphere implant A. Other arrangements are considered as well.

Although patient specific technology is suggested above, it is also considered to use a standard positioning device 10 (i.e., non-patient specific). In such a case, the device 10 would be attached to a trial version of the glenosphere that could easily be inserted on the baseplate. A pin could be driven through the superior hole of the device 10, for subsequent use to insert the permanent glenosphere implant A. As yet another embodiment, the positioning and orienting of the guide pin C could be navigated, for subsequent use of a standard positioning device 10.

According to another embodiment shown in Figs. 7A to 7D, a standard positioning device 10" is shown in greater detail, and has many features in common with the positioning devices 10 and 10' of Fig. 1 and Figs. 5A-5D, respectively, whereby like components will bear like reference numerals. The coupling body 12 of the positioning device 10" also has a C-shape, with a first portion being the arcuate member 20 defining a sphere-matching surface 21 forming a concavity for conforming contact with the glenosphere implant A, as shown in Figs. 7C and 7D. The second portion is the inward flange 22 located at a bottom edge of the first portion 20. The inward flange 22 abuts against the shoulder of the underside A1 of the glenosphere implant A, as seen in Fig. 5D. Again, the combination of the first portion and the second portion of the positioning device 10" allow it to be releasably coupled to the glenosphere implant A. Also like the positioning device 10', to further assist in the releasable coupling between the positioning device 10" and the glenosphere implant A, the C shape of the coupling body 12 may surround the glenosphere implant A over more than 180 degrees. In Fig. 5B, the C shape is shown as being more than a half circle for this purpose.

Referring to Figs. 7A and 7D, the inward flange 22 has a joint edge surface 30 precisely shaped and dimensioned to form a sliding joint with a circumferential surface B2 at a base of the baseplate B (Fig. 7D). The circumferential surface B2 of the baseplate B may be frusto-conical, quasi-cylindrical or cylindrical, and an axis thereof is normal to the plane B1 of the baseplate B. It may therefore be desired to align the positioning device 10" with the baseplate B to ensure that the implant A is correctly assembled to the baseplate B. Therefore, by using the joint edge surface 30 as complementary to the circumferential surface B2 of the baseplate B, an operator may be guided in properly aligning the implant A/positioning device 10" by the positioning device 10" being blocked from moving downwardly unless correctly aligned with the baseplate B. In an embodiment, the circumferential surface B2 is a section of a cone being larger than a cone of the morse taper between the baseplate B and the socket of the implant A.

A block 31 also projects upwardly from the coupling body 12 of the positioning device 10". The block 31 may be used with pliers or other tool, for removal of the positioning device 10" from the implant A once assembled to the baseplate B. Although not shown, it is considered to provide connectors for impactor tool D, and threaded hole 25 in the block 31. To releasably couple the positioning device 10" to the glenosphere implant A, the positioning device 10" is made of a material capable of elastic deformation when deformed to be coupled to the glenosphere implant A, such a medical-grade polymer or metal. In an embodiment, the positioning device 10" is a monolithic component. However, the rigidity and structural integrity of the positioning device 10", as the inward flange 22 must keep its shape for the joint edge surface 30 to form a reliable joint with the circumferential surface B2 of the baseplate B.

The method described above with reference to Figs. 2 and 3 does not use the guide pin C, but instead relies on the complementary engagement between the positioning device 10" and the baseplate B.

The positioning device 10/10'/10" may be provided as an installation kit with at least the glenosphere implant A, and possibly with the baseplate B, guide pin C and impactor tool interface D, along with other tools such as fasteners, etc.

## Claims

1. A glenosphere-implant positioning device (10, 10') for positioning a glenosphere implant (A) in a desired implanting orientation of the glenosphere implant (A) or to an implanted baseplate (B), the glenosphere implant (A) having a spherical portion and an underside portion (A1), the glenosphere-implant positioning device (10, 10') comprising :
a coupling body (12) having at least a first portion adapted to contact the spherical portion of the glenosphere implant (A), and a second portion adapted to contact the underside portion (A1) of the glenosphere implant (A), such that the coupling body (12) is configured to be releasably coupled to the glenosphere implant (A); and
a guide (14) connected to the coupling body (12) and configured to be slidingly engaged to a guide pin (C) representative of the desired implanting orientation.

2. The glenosphere-implant positioning device (10, 10') according to claim 1, wherein the coupling body (12) has a C shape.

3. The glenosphere-implant positioning device (10, 10') according to claim 2, wherein the first portion of the coupling body (12) is an arcuate member (20) having an inward sphere-matching surface (21) configured to conformly contact the spherical portion of the glenosphere implant (A).

4. The glenosphere-implant positioning device (10, 10') according to claim 3, wherein the second portion is a flange (22) projecting inwardly from an edge of the arcuate member (20) .

5. The glenosphere-implant positioning device (10, 10') according to claim 4, wherein the guide (14) is an edge surface of the flange (22) configured to form a sliding joint with the periphery (B2) of the implanted baseplate (B).

6. The glenosphere-implant positioning device (10, 10') according to any one of claims 2 to 5, wherein the C shape is configured to be releasably coupled over more than 180 degrees of the glenosphere implant (A).

7. The glenosphere-implant positioning device (10, 10') according to any one of claims 1 and 2, wherein the guide (14) defines a channel (16) configured to be slidingly engaged to a guide pin (C), the glenosphere-implant positioning device (10, 10') further comprising a patient specific geometry between the coupling body (12) and the channel (16) based on a planned position and orientation between the guide pin (C) and the baseplate (B) upon which the glenosphere implant (A) is to be implanted.

8. The glenosphere-implant positioning device (10, 10') according to any one of claims 1 to 7, further comprising at least one connector (23) projecting from the coupling body (12) and configured for releasable engagement with an impactor tool portion (D) contacting the glenosphere implant (A).

9. The glenosphere-implant positioning device (10, 10') according to claim 8, wherein the at least one connector (23) is configured to align the impact tool portion (D) with the desired implanting orientation.

10. The glenosphere-implant positioning device (10, 10') according to any one of claims 1 to 9, wherein the glenosphere-implant positioning device (10, 10') is a monolithic component.

11. A kit comprising:
the glenosphere-implant positioning device (10, 10') according to any one of claims 1 to 10;
the baseplate (B) configured to be anchored to a bone; and
the glenosphere implant (A) for mating engagement with the baseplate (B), the glenosphere implant (A) configured to be releasably coupled to the glenosphere-implant positioning device (10, 10') in a known manner.

12. The kit according to claim 11, further comprising an impactor tool interface (D) adapted to be positioned directly against a surface of the glenosphere implant (A).

13. The kit according to claim 11, wherein the impactor tool interface (D) is adapted to be releasably connected to the glenosphere-implant positioning device (10, 10') such that an axis of an impactor tool is parallel to a normal to a plane (B1) of the baseplate (B).

## Patentansprüche

1. Glenosphärenimplantatpositioniervorrichtung (10, 10') zur Positionierung eines Glenosphärenimplantats (A) in einer gewünschten Implantierausrichtung des Glenosphärenimplantats (A) oder auf einer implantierten Basisplatte (B), wobei das Glenosphärenimplantat (A) einen sphärischen Abschnitt und einen Unterseitenabschnitt (A1) aufweist,
wobei die Glenosphärenimplantatpositioniervorrichtung (10, 10') Folgendes umfasst:
einen Kopplungskörper (12), der zumindest einen ersten Abschnitt, der ausgelegt ist, um den sphärischen Abschnitt des Glenosphärenimplantats (A) zu kontaktieren, und einen zweiten Abschnitt aufweist, der ausgelegt ist, um den Unterseitenabschnitt (A1) des Glenosphärenimplantats (A) zu kontaktieren, sodass der Kopplungskörper (12) konfiguriert ist, um lösbar an das Glenosphärenimplantat (A) gekoppelt zu werden; und
eine Führung (14), die mit dem Kopplungskörper (12) verbunden und konfiguriert ist, um gleitend mit einem Führungsstift (C) in Eingriff genommen zu werden, der die gewünschte Implantierausrichtung darstellt.

2. Glenosphärenimplantatpositioniervorrichtung (10, 10') nach Anspruch 1, wobei der Kopplungskörper (12) eine C-Form aufweist.

3. Glenosphärenimplantatpositioniervorrichtung (10, 10') nach Anspruch 2, wobei der erste Abschnitt des Kopplungskörpers (12) ein bogenförmiges Element (20) ist, das eine einwärtige Sphärenpassfläche (21) aufweist, die konfiguriert ist, um den sphärischen Abschnitt des Glenosphärenimplantats (A) konform zu kontaktieren.

4. Glenosphärenimplantatpositioniervorrichtung (10, 10') nach Anspruch 3, wobei der zweite Abschnitt ein Flansch (22) ist, der von einem Rand des bogenförmigen Elements (20) nach innen vorsteht.

5. Glenosphärenimplantatpositioniervorrichtung (10, 10') nach Anspruch 4, wobei die Führung (14) eine Randfläche des Flansches (22) ist, die konfiguriert ist, um eine Gleitverbindung mit dem Umfang (B2) der implantierten Basisplatte (B) zu bilden.

6. Glenosphärenimplantatpositioniervorrichtung (10, 10') nach einem der Ansprüche 2 bis 5, wobei die C-Form konfiguriert ist, um lösbar über mehr als 180 Grad des Glenosphärenimplantats (A) gekoppelt zu werden.

7. Glenosphärenimplantatpositioniervorrichtung (10, 10') nach einem der Ansprüche 1 und 2, wobei die Führung (14) einen Kanal (16) definiert, der konfiguriert ist, um gleitend mit einem Führungsstift (C) in Eingriff genommen zu werden, wobei die Glenosphärenimplantatpositioniervorrichtung (10, 10') ferner eine patientenspezifische Geometrie zwischen dem Kopplungskörper (12) und dem Kanal (16) basierend auf einer geplanten Position und Ausrichtung zwischen dem Führungsstift (C) und der Basisplatte (B) umfasst, auf der das Glenosphärenimplantat (A) zu implantieren ist.

8. Glenosphärenimplantatpositioniervorrichtung (10, 10') nach einem der Ansprüche 1 bis 7, ferner umfassend zumindest einen Anschluss (23), der von dem Kopplungskörper (12) vorsteht und für lösbaren Eingriff mit einem Stoßkörperwerkzeugabschnitt (D) konfiguriert ist, der das Glenosphärenimplantat (A) kontaktiert.

9. Glenosphärenimplantatpositioniervorrichtung (10, 10') nach Anspruch 8, wobei der zumindest eine Anschluss (23) konfiguriert ist, um den Stoßkörperwerkzeugabschnitt (D) auf die gewünschte Implantierausrichtung auszurichten.

10. Glenosphärenimplantatpositioniervorrichtung (10, 10') nach einem der Ansprüche 1 bis 9, wobei die Glenosphärenimplantatpositioniervorrichtung (10, 10') eine monolithische Komponente ist.

11. Kit, umfassend:
die Glenosphärenimplantatpositioniervorrichtung (10, 10') nach einem der Ansprüche 1 bis 10;
die Basisplatte (B), die konfiguriert ist, um an einem Knochen verankert zu werden; und
das Glenosphärenimplantat (A) für Passeingriff mit der Basisplatte (B), wobei das Glenosphärenimplantat (A) konfiguriert ist, um auf eine bekannte Weise lösbar an die Glenosphärenimplantatpositioniervorrichtung (10, 10') gekoppelt zu werden.

12. Kit nach Anspruch 11, ferner umfassend eine Stoßkörperwerkzeugschnittstelle (D), die ausgelegt ist, um direkt gegen eine Fläche des Glenosphärenimplantats (A) positioniert zu werden.

13. Kit nach Anspruch 11, wobei die Stoßkörperwerkzeugschnittstelle (D) ausgelegt ist, um lösbar mit der Glenosphärenimplantatpositioniervorrichtung (10, 10') verbunden zu werden, sodass eine Achse eines Stoßkörperwerkzeugs parallel zu einer Senkrechten zu einer Ebene (B1) der Basisplatte (B) ist.

## Revendications

1. Dispositif de positionnement d'implant de glénosphère (10, 10') destiné à positionner un implant de glénosphère (A) dans une orientation d'implantation souhaitée de l'implant de glénosphère (A) ou par rapport à une embase implantée (B), l'implant de glénosphère (A) ayant une partie sphérique et une partie de face inférieure (A1),
le dispositif de positionnement d'implant de glénosphère (10,10') comprenant :
un corps de couplage (12) ayant au moins une première partie adaptée pour venir en contact avec la partie sphérique de l'implant de glénosphère (A), et une deuxième partie adaptée pour venir en contact avec la partie de face inférieure (A1) de l'implant de glénosphère (A), de manière à ce que le corps de couplage (12) soit configuré pour être couplé de manière détachable à l'implant de glénosphère (A) ; et
un guide (14) connecté au corps de couplage (12) et configuré pour être mis en prise de manière coulissante avec un axe de guidage (C) représentatif de l'orientation d'implantation souhaitée.

2. Dispositif de positionnement d'implant de glénosphère (10,10') selon la revendication 1, dans lequel le corps de couplage (12) a une forme de C.

3. Dispositif de positionnement d'implant de glénosphère (10, 10') selon la revendication 2, dans lequel la première partie du corps de couplage (12) est un élément arqué (20) ayant une surface correspondant à une sphère vers l'intérieur (21) configurée pour venir en contact de manière conforme avec la partie sphérique de l'implant de glénosphère (A).

4. Dispositif de positionnement d'implant de glénosphère (10, 10') selon la revendication 3, dans lequel la deuxième partie est une bride (22) dépassant vers l'intérieur depuis un bord de l'élément arqué (20).

5. Dispositif de positionnement d'implant de glénosphère (10, 10') selon la revendication 4, dans lequel le guide (14) est une surface de bord de la bride (22) configurée pour former un joint coulissant avec la périphérie (B2) de l'embase implantée (B).

6. Dispositif de positionnement d'implant de glénosphère (10, 10') selon l'une quelconque des revendications 2 à 5, dans lequel la forme en C est configurée pour être couplée de manière détachable sur plus de 180 degrés de l'implant de glénosphère (A).

7. Dispositif de positionnement d'implant de glénosphère (10, 10') selon l'une quelconque des revendications 1 et 2, dans lequel le guide (14) définit un canal (16) configuré pour être mis en prise de manière coulissante avec un axe de guidage (C), le dispositif de positionnement d'implant de glénosphère (10, 10') comprenant en outre une géométrie spécifique au patient entre le corps de couplage (12) et le canal (16) sur la base d'une position et orientation prévues entre l'axe de guidage (C) et l'embase (B) selon lesquelles l'implant de glénosphère (A) doit être implantée.

8. Dispositif de positionnement d'implant de glénosphère (10, 10') selon l'une quelconque des revendications 1 à 7, comprenant en outre au moins un connecteur (23) dépassant depuis le corps de couplage (12) et configuré pour venir en prise de manière détachable avec une partie d'outil d'impacteur (D) en contact avec l'implant de glénosphère (A).

9. Dispositif de positionnement d'implant de glénosphère (10, 10') selon la revendication 8, dans lequel l'au moins un connecteur (23) est configuré pour aligner la partie d'outil d'impacteur (D) avec l'orientation d'implantation souhaitée.

10. Dispositif de positionnement d'implant de glénosphère (10, 10') selon l'une quelconque des revendications 1 à 9, dans lequel le dispositif de positionnement d'implant de glénosphère (10, 10') est un composant monolithique.

11. Kit comprenant :
le dispositif de positionnement d'implant de glénosphère (10, 10') selon l'une quelconque des revendications 1 à 10 ;
l'embase (B) configurée pour être ancrée à un os ; et
l'implant de glénosphère (A) destiné à venir en prise par accouplement avec l'embase (B), l'implant de glénosphère (A) configuré pour être couplé de manière détachable au dispositif de positionnement d'implant de glénosphère (10, 10') d'une manière connue.

12. Kit selon la revendication 11, comprenant en outre une interface d'outil d'impacteur (D) adaptée pour être positionnée directement contre une surface de l'implant de glénosphère (A).

13. Kit selon la revendication 11, dans lequel l'interface d'outil de l'impacteur (D) est adaptée pour être connectée de manière détachable au dispositif de positionnement d'implant de glénosphère (10, 10') de manière à ce qu'un axe d'un outil d'impacteur soit parallèle à un axe normal à un plan (B1) de l'embase (B).
